# EUROPÄISCHE PATENTSCHRIFT

(11) **EP 2 590 686 B1**
(45) Veröffentlichungstag und Bekanntmachung des Hinweises auf die Patenterteilung: **31.08.2016**
(21) Anmeldenummer: 11730585.4
(22) Anmeldetag: 05.07.2011
(51) Int. Cl.: A61L 2/20

(54) **VERFAHREN ZUR STERILISATION UND STERILISATIONSVORRICHTUNG**
METHOD FOR STERILISING AND STERILISING DEVICE
PROCÉDÉ DE STÉRILISATION ET DISPOSITIF DE STÉRILISATION

(30) Priorität: 05.07.2010 DE 102010026104
(43) Veröffentlichungstag der Anmeldung: 15.05.2013
(73) Patentinhaber: Fresenius Medical Care Deutschland GmbH, 61352 Bad Homburg (DE)
(72) Erfinder: FEHR, Thorsten, 66679 Losheim am See (DE); HAUPERT, Claus, 66809 Nalbach (DE); KUGELMANN, Franz, 66606 St. Wendel/Bliesen (DE); SIMON, Nicole, 66839 Schmelz (DE)
(74) Vertreter: Herrmann, Uwe
(86) Internationale Anmeldenummer: PCT/EP2011/003342
(87) Internationale Veröffentlichungsnummer: WO 2012/003967

(56) Entgegenhaltungen:
- EP-A1- 1 159 971
- WO-A1-00/66186
- WO-A1-93/17726
- WO-A1-03/039607
- WO-A1-2005/031220
- US-A- 4 203 943
- US-A- 5 556 607
- US-A1- 2002 085 950
- S.S. BLOCK: 31. Dezember 2001 (2001-12-31), XP002658107, Seiten 751-753, Seite 751, rechte Seite, markierte Passage; Abbildung 38.2

## Beschreibung

Die vorliegende Erfindung betrifft ein Verfahren und eine Vorrichtung zur Sterilisation eines oder mehrerer Gegenstände.

Die heute zur Anwendung kommenden Sterilisationsverfahren, wie z. B. die sogenannte ETO-Sterilisation, die Gamma-Bestrahlung oder auch die Dampfsterilisation sind für bestimmte Sterilisationsvorgänge ungeeignet, da sie teilweise mit hohen Temperaturen arbeiten und so gegebenenfalls den zu sterilisierenden Gegenständen schaden, zu hohe Kosten verursachen und/oder nicht umweltverträglich sind. Dies gilt vor allem für die genannte ETO-Sterilisation.

Des Weiteren ist es bekannt, die Sterilisation unter Zuhilfenahme von Ozon durchzuführen. Dies hat insbesondere gegenüber der ETO-Sterilisation den Vorteil, dass z. B. das Ozon und H₂O₂ nach erfolgter Sterilisation über einen Katalysator zu Sauerstoff und Wasser umgesetzt und damit unschädlich gemacht werden kann. Sterilisationsverfahren, die unter dem Einsatz von Ozon arbeiten, sind aus dem Stand der Technik ebenfalls bekannt.

So ist es beispielsweise aus der EP 1 175 230 B1 sowie aus der EP 1 455 843 B1 bekannt, einen Sterilisationsvorgang derart vorzunehmen, dass nach der Evakuierung einer Sterilisationskammer die Befeuchtung der in der Sterilisationskammer befindlichen Atmosphäre und sodann die Befüllung mit Ozon erfolgt, wodurch der Sterilisierungsvorgang eingeleitet wird. Diese Behandlungsschritte werden wiederholt, bevor die Sterilisationskammer gespült und schließlich geöffnet wird, so dass die sterilisierten Gegenstände entnommen werden können. Die aus den genannten Druckschriften bekannten Vorrichtungen und Verfahren eignen sich in der Praxis wegen ihres Aufbaus und der Verfahrensdurchführung nur für kleine Gebinde, wie beispielsweise Operationsbesteck in kleinen Behältern.

Aus der US 4,203,943 ist die Sterilisation mittels Wasserdampf bekannt, dem Zusatzagentien, wie beispielsweise Alkohol, Ketone oder Ether beigefügt werden können. Die US 5,556,607 offenbart die Sterilisation mittels eines mehrere Komponenten enthaltenden Gemisches, wobei eine Komponente durch Wasser gebildet wird. Aus der Druckschrift "Sterilization Process Utilizing Low-Temperature Water" (S.S. Block, 31.12.2001, XP000002658107, pp. 751-753) ist ein Sterilisationsverfahren bekannt, bei dem zwei Drücke zur Anwendung kommen. Das Sterilisationsmedium wird durch eine wässrige Wasserstoffperoxid-Lösung gebildet. Die WO 03/039607 A1 offenbart ein Sterilisationsverfahren, bei dem als Sterilisationsmedium eine Mischung aus Ozon und Sauerstoff verwendet wird. Die WO 2005/031220 A1 betrifft ein Sterilisationsverfahren, bei dem zwei Druckbereiche durchlaufen werden. Der erste Bereich wird durch die Einleitung von Wasserdampf und der zweite Bereich durch die Einleitung von Ozon erhalten. Die US 2002/0085950 A1 betrifft die Durchführung eines Sterilisationsprozesses unter Einführung eines Sauerstoff/Ozon-Gemisches in einer Sterilisationskammer.

Es ist daher die Aufgabe der vorliegenden Erfindung, ein Verfahren und eine Vorrichtung der eingangs genannten Art in vorteilhafter Weise weiterzubilden, insbesondere dahingehend, dass ein Sterilisationsverfahren und eine Vorrichtung hierzu geschaffen wird, mittels dessen bzw. mittels derer eine sichere und zuverlässige Sterilisation auch schwer zur sterilisierender Gegenstände wie z. B. langer und/oder einseitig verschlossener Schlauchsysteme möglich ist.

Erfindungsgemäß wird diese Aufgabe durch ein Verfahren mit den Merkmalen des Anspruchs 1 gelöst. Danach ist vorgesehen, dass bei einem Verfahren zur Sterilisation wenigstens eines Gegenstandes der Gegenstand einem Sterilisationsmittel ausgesetzt wird, wobei wenigstens ein erster und wenigstens ein zweiter Stützdruck angelegt werden und wobei durch das Sterilisationsmittel bei dem ersten Stützdruck eine Sterilisierung wenigstens eines ersten Bereiches des Gegenstandes und bei dem zweiten Stützdruck eine Sterilisierung wenigstens eines zweiten Bereiches des Gegenstandes erfolgt.

Dadurch ergibt sich insbesondere der Vorteil, dass eine ETO-freie, zuverlässige und sichere Sterilisation erreicht werden kann. Der Stützdruck kann durch die Zugabe eines Stützgases in einer Sterilisationskammer erzeugt werden, wodurch das in die Sterilisationskammer eingeleitete Sterilisationsmittel auf den zu sterilisierenden Gegenstand einwirkt und diesen bereichsweise sterilisiert.

Insbesondere ist möglich, dass der Gegenstand ein irgendwie geartetes Disposable ist, das durch seine Geometrie für das Sterilisationsmittel schwer zugängliche Bereiche, wie z. B. Lumen oder dergleichen, aufweist.

Der Gegenstand kann ein Schlauchsystem, insbesondere ein medizinisches Schlauchsystem bzw. ein sogenanntes Schlauchset etwa zur Anwendung in der Hämodialyse sein. Vorzugsweise wird in Abhängigkeit vom Stützdruck dieses Schlauchsystem bereichsweise sterilisiert. Je nach anliegendem Stützdruck kann das Sterilisationsmittel in das Lumen des Schlauchsystems hinein transportiert werden.

Es ist insbesondere möglich, lange Schlauchsysteme bzw. Schlauchsets sterilisieren zu können, selbst wenn diese in gasdurchlässigen Umverpackungen eingebracht sind und/oder einseitig verschlossen sind.

Vorteilhafterweise kann somit verhindert werden, dass bei hohen Druckwerten das Sterilisationsmittel in der Mitte z. B. von zu sterilisierenden Schlauchsystemen aufkonzentriert wird, während es an den jeweiligen Enden durch das Stützgas verdünnt wird. Es wird möglich, dem Umstand Rechnung zu tragen, dass bei Anwendung eines niedrigen Stützdrucks eher die äußeren Enden des zu sterilisierenden Gegenstandes erreicht werden, etwa bei einem Schlauchsystem die Schlauchenden. Durch die Anwendung von unterschiedlichen Stützdrücken wird es nunmehr möglich, gezielt alle Bereiche des zu sterilisierenden Gegenstandes mit dem Sterilisationsmittel zu befüllen. Besonders vorteilhaft ist, dass die Sterilisation auch in allen Bereichen eines geschlossenen Lumens möglich ist.

Ein weiterer Vorteil besteht darin, dass eine sichere Alternative zur sogenannten Gammasterilisation bereitgestellt ist. Insbesondere kann somit verhindert werden, dass es in Folge der Bestrahlung zu einer Materialveränderung, schlimmstenfalls zu einer Materialschädigung des zu sterilisierenden Gegenstandes kommt.

Es ist ferner denkbar, dass das Sterilisationsmittel zumindest teilweise Ozon und/oder Wasserstoffperoxid oder eines oder mehrere der Reaktionsprodukte dieser Substanzen enthält. Beispielsweise wird bei der Verwendung von Wasserstoffperoxid als Sterilisationsmittel eine Vorwärmung von Sterilisationskammer und Sterilisationsgut vorgenommen. Bei der Verwendung von Ozon als Sterilisationsmittel ist dies nicht zwingend erforderlich. Grundsätzlich ist es beispielsweise denkbar, nur Wasserstoffperoxid oder nur Ozon als Sterilisationsmittel zu verwenden.

Erfindungsgemäß wird der wenigstens eine erste und/oder wenigstens eine zweite Stützdruck durch Einleitung eines Stützgases in eine Sterilisationskammer erzeugt.

Je nach Anwendungszweck und Beschaffenheit des zu sterilisierenden Gegenstandes kann vorgesehen sein, dass der wenigstens eine erste und/oder der wenigstens eine zweite Stützdruck besonders vorteilhaft unter Atmosphärendruck liegt. Alternativ kann vorgesehen sein, dass der wenigstens eine erste und/oder der wenigstens eine zweite Stützdruck bei oder über Atmosphärendruck liegt.

Erfindungsgemäß ist vorgesehen, dass das Stützgas zumindest teilweise ein Inertgas und/oder Luft enthält, vorzugsweise sterile Luft enthält oder ist.

Es ist denkbar, dass in einem ersten Schritt der zu sterilisierende Gegenstand in eine Sterilisationskammer eingelegt wird, in einem zweiten Schritt die Sterilisationskammer evakuiert wird, in einem dritten Schritt die Sterilisationskammer befeuchtet und Sterilisationsmittel in die Sterilisationskammer eingeführt wird, in einem vierten Schritt der erste Stützdruck angelegt wird, in einem fünften Schritt der erste Stützdruck für einen Zeitpunkt oder eine Zeitspanne gehalten wird, in einem sechsten Schritt die Schritte zwei bis fünf ein- oder mehrfach wiederholt werden und in einem siebten Schritt der wenigstens eine zweite Stützdruck angelegt wird.

Bei der Druckzunahme kommt es zum Ausfallen der in der Atmosphäre gelösten Flüssigkeit. Hierdurch entsteht eine nebelartige Atmosphäre, die als Aerosol zusätzlich Wirkung zeigt. Diese kann durch schnelles Abkühlen eines gesättigten Gases oder hier auch durch schnelles Erhöhen des Drucks erzeugt werden, wobei es sich bei der letzteren Variante um eine bevorzugte Ausgestaltung der Erfindung handelt.

Ferner ist möglich, dass im siebten Schritt die Schritte zwei bis fünf oder zwei bis sechs mit dem zweiten Stützdruck durchgeführt werden.

Vorteilhafterweise kann weiter vorgesehen sein, dass in zumindest einem weiteren Schritt ein oder mehrere weitere Stützdrücke angelegt werden und dass die Schritte zwei bis fünf oder zwei bis sechs mit den weiteren Stützdrücken entsprechend durchgeführt werden.

Darüber hinaus ist es denkbar, dass das Befeuchten der Sterilisationskammer dadurch erfolgt, dass eine Flüssigkeit, insbesondere ein Gemisch enthaltend Wasser und Wasserstoffperoxid, durch Anlegen eines Vakuums, insbesondere durch das Evakuieren zum Verdampfen gebracht wird und die verdampfte Flüssigkeit der Sterilisationskammer zugeführt wird. Von Vorteil ist, dass dieser so erzeugte Dampf enthaltend Wasserstoffperoxid jedenfalls keine hohe Temperatur erreicht, die das Sterilisationsgut schädigen könnte. Es handelt sich also um einen vergleichweise kalten Dampf.

Außerdem kann vorgesehen sein, dass der erste Stützdruck mindestens 500 mbar abs und/oder der zweite Stützdruck mindestens 50 mbar abs beträgt.

Weiterhin ist möglich, dass ein dritter Stützdruck angelegt wird, wobei der dritte Stützdruck vorzugsweise bis zu 50 mbar abs beträgt und/oder dem Begasungsdruck ohne zusätzliches Einleiten eines Stützgases entspricht, wobei hierdurch der vierte Schritt, also das Anlegen des Stützdruckes, bereits durch Schritt drei, also das Einführen des Sterilisationsmittels in die Sterilisationskammer, durchgeführt wird.

Insbesondere hat jeder Druck bzw. Stützdruck vorzugsweise eine Obergrenze, die 1100 mbar beträgt.

Weiter ist möglich, dass das Sterilisationsmittel in die Sterilisationskammer in Form eines Aerosols eingeführt wird.

Es kann vorgesehen sein, dass das Aerosol durch Dampf enthaltend Wasserstoffperoxid gebildet ist. Beispielsweise kann vorgesehen sein, dass das als Sterilisationsmittel verwendete Wasserstoffperoxid als Dampf in die Sterilisationskammer geleitet wird.

Denkbar ist auch, dass das Aerosol durch Einleiten von Ozon in eine Flüssigkeit entsteht, die Wasser und/oder Wasserstoffperoxid enthält. Denkbar ist es beispielsweise, Ozongas durch eine sogenannte Laskindüse zu leiten. Diese Düse ist mit ihrer Auslassöffnung unterhalb des Pegels einer Flüssigkeit angeordnet, die aus Wasserstoffperoxid und/oder Wasser besteht oder eine oder mehrerer dieser Substanzen enthält. In der entstehenden, aus Ozon bestehenden Gasblase befinden sich kleine Flüssigkeitstropfen, die insgesamt eine sehr große Oberfläche haben, die direkt mit dem Ozongas in Berührung stehen. Der Kontakt des Ozons mit Wasser und/oder mit Wasserstoffperoxid führt zur Aktivierung der Flüssigkeiten durch eine Reaktion zwischen Wasser und/oder Wasserstoffperoxid und dem Ozon. Diese Reaktion führt zum Aufspalten des Wassers bzw. Wasserstoffperoxids und dann zur Radikalbildung. Die Hydroxidradikale OH⁻ sind reaktiv und aggressiv und bewirken durch Töten von Mikroorganismen den gewünschten Sterilisationserfolg.

Aufgrund der vergleichsweise großen Kontaktfläche zwischen der Flüssigkeit mit dem Ozongas werden viele der genannten Reaktionen angeregt, so dass eine entsprechend hohe Ausbeute an OH⁻-Radikalen vorliegt.

In weiterer Ausgestaltung der Erfindung ist vorgesehen, dass das Einführen eines Sterilisationsmittels in die Sterilisationskammer bei einem Druck in der Sterilisationskammer erfolgt, der unterhalb des Atmosphärendruckes liegt.

Diese Vorgehensweise ermöglicht es, den Aerosolbehälter und/oder -generator während des gesamten Betriebes stets bei einem Druckwert zu halten, der unterhalb des Atmosphärendruckes liegt. Diese Vorgehensweise bringt den Vorteil mit sich, dass ein Nachfüllen des Aerosolbehälters durch Wasserstoffperoxid bzw. Wasser besonders einfach ist, da lediglich ein Dosierventil geöffnet werden muss, durch das dann die entsprechende Flüssigkeit nachgezogen werden kann.

Darüber hinaus ist denkbar, dass das Halten des Stützdrucks, vorzugsweise gemäß dem fünften Schritt, also des Haltens des Stützdruckes für einen Zeitpunkt oder eine Zeitspanne, für eine Zeitspanne erfolgt, deren Dauer im Bereich < 20 Minuten, vorzugsweise < 10 Minuten und besonders bevorzugt < 5 Minuten ist.

Vorteilhafterweise kann weiter vorgesehen sein, dass die Zeitspanne zwischen zwei aufeinander erfolgenden Anlegeschritten, vorzugsweise gemäß viertem Schritt, also dem Anlegen des Stützdruckes, im Bereich < 20 Minuten, vorzugsweise < 15 Minuten und besonders bevorzugt < 10 Minuten ist.

Denkbar ist weiter, dass nach dem letzten Verfahrensschritt, insbesondere dem siebten Schritt, also dem Anlegen des wenigstens einen zweiten Stützdruckes, bzw. nach Abschluss der Sterilisation das Spülen und Entgasen der Sterilisationskammer durchgeführt wird, wobei das Spülen und Entgasen vorzugsweise mehrfach durchgeführt wird.

Denkbar ist es, diese Spül- und Entgasungsphase durch Wiederholungen von Evakuierungen und Belüftungen auszugestalten. Ebenfalls ist es vorteilhaft denkbar, eine Trockenphase vorzusehen, die vorzugsweise im evakuierten Zustand der Sterilisationskammer durchgeführt wird. Sodann kann der Sterilisationsvorgang abgeschlossen werden.

Möglich ist es, dass die Abfolge der Schritte zwei bis fünf 15 bis 20 Minuten, vorzugsweise 18 Minuten dauert.

Des Weiteren betrifft die Erfindung eine Sterilisationsvorrichtung mit den Merkmalen des Anspruchs 3. Danach ist vorgesehen, dass die Sterilisationsvorrichtung wenigstens eine Sterilisationskammer für die Aufnahme wenigstens eines zu sterilisierenden Gegenstandes, wenigstens ein erstes Mittel zur Zuführung und/oder Abführung eines Sterilisationsmittels und wenigstens ein zweites Mittel aufweist, mittels dessen wenigstens ein erster und wenigstens ein zweiter Stützdruck anlegbar ist und wobei bei dem ersten Stützdruck das Sterilisationsmittel eine Sterilisierung eines ersten Bereiches des Gegenstandes durchführbar und bei dem zweiten Stützdruck eine Sterilisierung eines zweiten Bereiches des Gegenstandes durchführbar ist.

Vorteilhafterweise kann das erste Mittel eine Zuführleitung sein, in der wenigstens ein Absperrventil vorhanden ist. Denkbar ist, dass die Zuführleitung zugleich eine Abführleitung ist. Grundsätzlich ist jedoch vorteilhaft möglich, die Zuführleitung und die Abführleitung getrennt voneinander auszuführen. Das zweite Mittel kann eine Stützgaszuführung mit entsprechender Druckregulierung aufweisen, wobei vorteilhafterweise die Zuführleitung für das Stützgas wenigstens ein Ventil aufweist. Über die Druckregulierung können unterschiedliche Stützdrücke eingestellt werden.

Erfindungsgemäß weist die Sterilisationsvorrichtung wenigstens ein Steuerungs- und/oder Regelungsmittel auf, mittels dessen der Sterilisationsvorgang steuerbar und/oder regelbar und/oder überwachbar ist, vorzugsweise halbautomatisch und/oder vollautomatisch steuerbar und/oder regelbar und/oder überwachbar ist. Dieses Steuerungs- und/oder Regelungsmittel kann beispielsweise ein Teil der zentralen Steuerungs- und/oder Regelungseinheit der Sterilisationsvorrichtung sein.

Ferner ist möglich, dass mittels des zweiten Mittels der erste und/oder der zweite Stützdruck mit einem Druck anlegbar ist, der besonders vorteilhaft unter Atmosphärendruck liegt. Alternativ ist auch denkbar, dass mittels des zweiten Mittels der erste und/oder der zweite Stützdruck mit einem Druck anlegbar ist, der bei oder über Atmosphärendruck liegt.

Erfindungsgemäß ist vorgesehen, dass mit der Sterilisationsvorrichtung ein Verfahren nach einem der Ansprüche 1 durchführbar ist. Es ist vorgesehen, dass die Durchführung des Verfahrens durch das Steuerungs- und/oder Regelungsmittel steuerbar und/oder regelbar und/oder überwachbar ist, vorzugsweise halbautomatisch und/oder vollautomatisch steuerbar und/oder regelbar und/oder überwachbar ist.

Weitere Einzelheiten und Vorteile sollen nun anhand eines in der Zeichnung dargestellten Ausführungsbeispiels näher erläutert werden. Es zeigen:
- Figur 1:: eine schematische Darstellung einer Sterilisationsvorrichtung gemäß der vorliegenden Erfindung;
- Figur 2:: eine Darstellung des Druckverlaufes in der Sterilisationskammer während eines Sterilisationszyklus; und
- Figur 3:: eine schematische Darstellung eines medizinisches Schlauchsets mit Angabe der unterschiedlichen Sterilisationsbereiche.

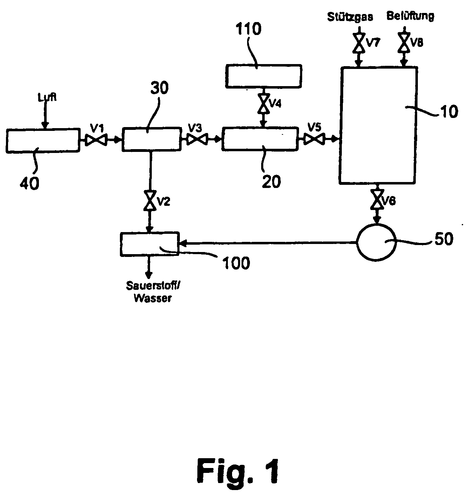

Figur 1 zeigt ein Blockschaltbild für ein Ausführungsbeispiel einer Sterilisationsvorrichtung gemäß der vorliegenden Erfindung. Mit dem Bezugszeichen 10 ist eine Sterilisationskammer gekennzeichnet, die beispielsweise ein Volumen von wenigstens 1 m³ haben kann sowie eine Tür, die sowohl zum Be- als auch zum Entladen dient. Die zu sterilisierenden Gegenstände können beispielsweise auf Stahlkörben auf einer oder mehreren Ebenen in die Sterilisationskammer 10 eingebracht werden. Je nach Produktgröße können 150 bis 200 Produkte gleichzeitig sterilisiert werden. Der Sterilisationskammer 10 vorgeschaltet ist ein Aerosolbehälter 20 oder Verdampfer 20.

Wie dies weiter aus Figur 1 hervorgeht, ist dem Ozongenerator 30 ein Sauerstoffgenerator 40 vorgeschaltet, in dem 95%iger Sauerstoff aus der Umgebungsluft gewonnen wird. Hierzu dient beispielsweise ein Molekularsieb bzw. Zeolith. In dem Ozongenerator 30 wird der Sauerstoff in Ozon umgewandelt, was beispielsweise durch eine dielektrische Barriereentladung erfolgen kann.

Wenn das erfindungsgemäße Verfahren ausschließlich mit Wasserstoffperoxid durchgeführt wird, kann die Figur 1 gezeigte Vorrichtung entsprechend abgewandelt ausgeführt sein. Beispielsweise kann dann auf den Ozongenerator 30 verzichtet werden. Statt dessen kann beispielsweise ein Reservoir bzw. Tank für das Wasserstoffperoxid oder allgemein eine Wasserstoffperoxidzufuhr vorgesehen sein. Das Wasserstoffperoxid wird vorzugsweise in Dampfform, etwa als durch Evakuieren verdampfte Flüssigkeit, in die Sterilisationskammer 10 eingebracht.

Mit dem Bezugszeichen 100 in Figur 1 ist ein Katalysator gekennzeichnet, der geeignet ist, das Sterilisationsmittel nach dessen Gebrauch, insbesondere Wasserstoffperoxid und/oder Ozon bzw. deren Reaktionsprodukte zu zersetzen. Als Zersetzungsprodukte entstehen dann lediglich Wasser und Sauerstoff. Bei dem Katalysator 100 kann es sich beispielsweise um Mangandioxid handeln.

Zur Erzeugung des gewünschten Vakuums in der Sterilisationskammer 10 ist dieser eine Vakuumpumpe 50 nachgeschaltet, die bei geöffnetem Ventil V6 und bei in Betrieb befindlicher Vakuumpumpe 50 zu einem Evakuieren der Sterilisationskammer 10 führt. Über das Ventil V7 wird als Stützgas sterile Luft in die Sterilisationskammer 10 eingeleitet. Mit dem Bezugszeichen 110 ist schließlich eine Einheit angedeutet, die der Zudosierung von Wasser und/oder Wasserstoffperoxid in den Aerosolbehälter 20 oder Verdampfer 20 dient.

Der Sterilisationsvorgang gestaltet sich im Einzelnen wie folgt:
Nach dem Einlegen des oder der Gegenstände in die Sterilisationskammer 10 wird in der Sterilisationskammer 10 ein Vakuum erzeugt, wozu nur das Ventil V6 gemäß Figur 1 geöffnet und alle weiteren Ventile V1, V2, V3, V4, eventuell V5, V7 und V8 geschlossen werden. Die Vakuumerzeugung führt zu einer Reduzierung des Kammerdruckes.

Durch das Evakuieren der Sterilisationskammer 10 mittels der Vakuumpumpe 50 im Schritt 200 fällt der Druck in der Kammer 10 vorzugsweise auf einen Wert < 10 mbar ab.

Um zu Befeuchten kann z. B. das Ventil V5 in der Leitung zwischen dem Aerosolbehälter 20 oder Verdampfer 20 und der Sterilisationskammer 10 geöffnet werden, so dass es zu einem Verdampfen der Flüssigkeit in dem Aerosolbehälter 20 kommt, sofern deren Dampfdruck unterschritten wird. Wird der Dampfdruck der Flüssigkeit, d. h. beispielsweise der Mischung aus Wasser und Wasserstoffperoxid, in dem Aerosolbehälter unterschritten, fängt diese an zu verdampfen und gelangt auf diese Weise in die Sterilisationskammer 10. Die Vakuumpumpe 50 wird somit nicht nur zum Evakuieren, sondern auch zum Verdampfen der Flüssigkeit in dem Aerosolbehälter 20 genutzt.

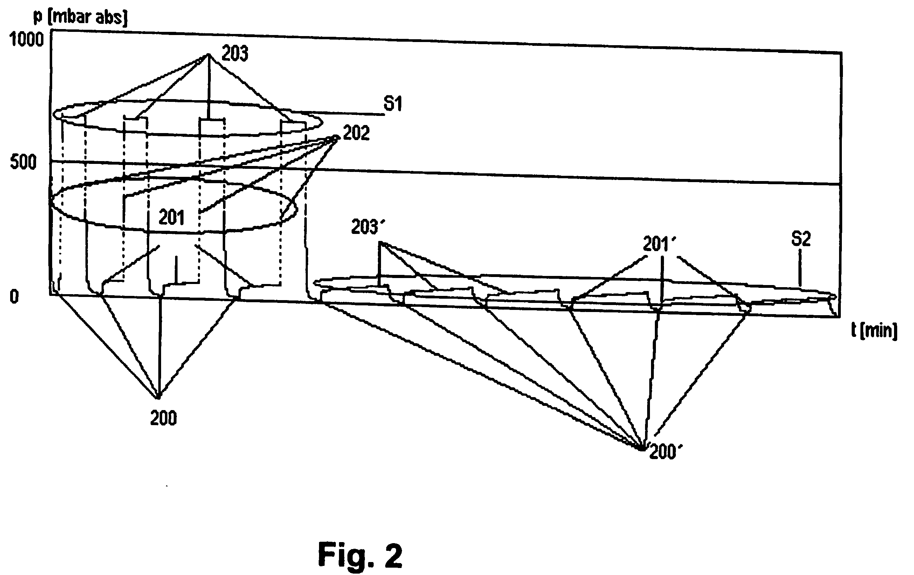

Gleichzeitig mit dem Schritt des Befeuchtens wird aktiv Sterilisationsmittel in die Sterilisationskammer 10 eingebracht. Diese Verfahrensschritt ist in Figur 2 mit dem Bezugszeichen 201 gekennzeichnet. Vorteilhaft wird hier beispielsweise als Sterilisationsmittel Wasserstoffperoxid als Dampf in die Sterilisationskammer eingeleitet.

Alternativ kann durch den Kontakt des Ozons mit Wasser und/oder Wasserstoffperoxid es zur Entstehung von Hydroxidradikalen kommen, die mit dem Aerosol in die Sterilisationskammer 10 gelangen und dort zum Sterilisationsprozess beitragen bzw. die maßgeblichen Sterilisationsmittel darstellen.

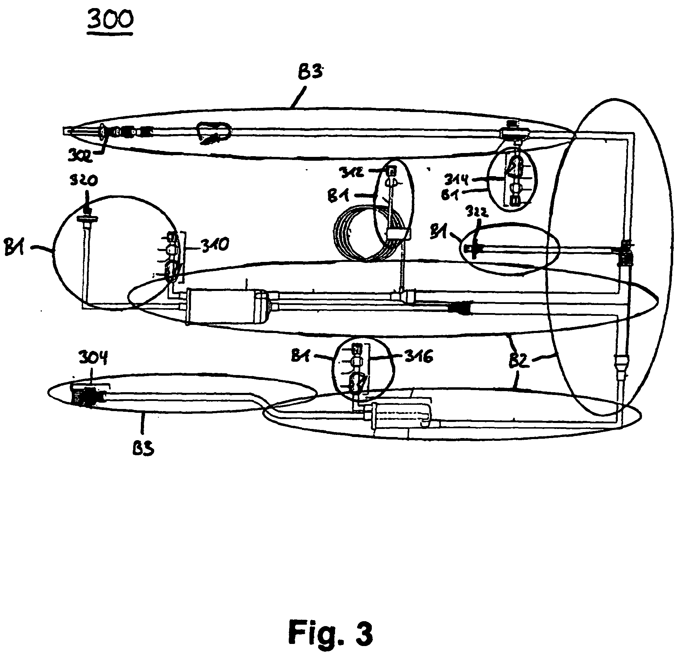

Während des Verfahrensschrittes 201 gemäß Figur 3 sind die Ventile V3 und V5 oder V4 und V5 geöffnet und alle weiteren Ventile geschlossen. Das ozonhaltige Aerosol gelangt aufgrund des Druckgefälles zwischen Ozongenerator 30 und Sterilisationskammer 10 in die Sterilisationskammer 10.

Dies gilt entsprechend für den Wasserstoffperoxiddampf, wenn Wasserstoffperoxid an Stelle von Ozon als Sterilisationsmittel verwendet wird.

Dadurch kommt es zu einem Druckanstieg, wie dies aus Figur 2 ersichtlich ist. Dieser Druckanstieg ist ein Maß für die Menge des Sterilisationsmittels in der Sterilisationskammer 10. Hierbei wird die Konzentration auf einen für das zu sterilisierende Gut optimalen Wert eingestellt.

Nach dem Einführen des Sterilisationsmittels wird der Druck in der Sterilisationskammer 10 mittels eines Stützgases über das Ventil V7 erhöht, wie dies in Schritt 202 gemäß Figur 2 dargestellt ist. In dem dort dargestellten Ausführungsbeispiel wird der Druck auf einen ersten Stützdruck von ca. 500 mbar abs erhöht. Dieses Stützgas gelangt dadurch in die Sterilisationskammer 10, dass das Ventil 7 gemäß Figur 1 für eine vorbestimmte Zeitspanne oder bis zum Erreichen eines bestimmten Druckwertes geöffnet wird. Während dieses Verfahrensschrittes sind alle weiteren Ventile der Vorrichtung geschlossen. Alternativ kann V5 auch geöffnet sein.

Die Sterilisationsphase, bei der der erste Stützdruck angelegt wird, wird als erste Sterilisationsphase S1 bezeichnet.

Wie dies weiter aus Figur 2 hervorgeht, wird der erste Stützdruck in der Sterilisationskammer 10 für eine bestimmte Zeitspanne gehalten. Dieser Verfahrensschritt ist in Figur 2 mit dem Bezugszeichen 203 gekennzeichnet und kann beispielsweise bei einer Minute bzw. im Minutenbereich liegen.

Nach dem Halten des Stützdruckes für eine bestimmte Zeitspanne gemäß Schritt 203 in Figur 2 wird dieser Prozessabschnitt bestehend aus den Schritten 200 bis 203 insgesamt viermal vorgenommen, d. h. es wird evakuiert, dann befeuchtet, und das Sterilisationsmittel eingeführt und sodann wieder der erste Stützdruck erzeugt und für eine bestimmte Zeitspanne gehalten.

Nach der viermaligen Durchführung der Schritte 200 bis 203 wird die erste Sterilisationsphase S1 beendet und mit der zweiten Sterilisationsphase S2 fortgefahren. In der Sterilisationsphase S2 wird der zweite Stützdruck angelegt, der hier 50 mbar abs beträgt, und die Schritte 200', 201', 202' und 203' werden insgesamt sechsmal vorgenommen. Dabei entsprechen die Schritte 200' bis 203' den Schritten 200 bis 203 aus der Sterilisationsphase S1, d. h. es wird evakuiert, dann befeuchtet und das Sterilisationsmittel eingeführt und sodann wieder der zweite Stützdruck erzeugt, mit dem Unterschied, dass in der Sterilisationsphase S2 der zweite Stützdruck 50 mbar abs beträgt.

Aufgrund der Tatsache, dass das Befüllen der Sterilisationskammer 10 mit Sterilisationsmittel gemäß Schritt 201, 201' 201" beendet wird, wenn in der Sterilisationskammer 10 noch ein erheblicher Unterdruck herrscht, kann grundsätzlich erreicht werden, dass in dem Aerosolgenerator 20 stets ein Unterdruck herrscht. Dies ermöglicht es, dass durch Öffnen des Ventils V4 aus einem Reservoir 110 ohne Weiteres Flüssigkeit, d. h. Wasser, Wasserstoffperoxid und/oder eine Mischung aus beiden Substanzen nachgezogen werden kann.

Wie dies weiter aus Figur 1 hervorgeht, steht die Pumpe 50 auslassseitig mit dem Katalysator 100 in Verbindung, so dass das auf der Druckseite der Pumpe 50 anfallende Medium in dem Katalysator 100 zersetzt werden kann.

Grundsätzlich ist denkbar, die Sterilisationsphasen S1 und S2 beispielsweise zu wiederholen und/oder zu variieren bzw. weitere Sterilisationsphasen mit anderen Stützdrücken anschließend an die in Figur 2 gezeigten Sterilisationsphasen durchzuführen.

Danach schließt sich in dem hier dargestellten Ausführungsbeispiel das Spülen und Entgasen der Sterilisationskammer 10 durch mehrmaliges Evakuieren und Belüften an. Diese Phase ist in Figur 2 nicht näher dargestellt.

Während der Spül- und Entgasungsphase kommt es mehrfach zu einem Evakuieren sowie zu einem Belüften der Sterilisationskammer 10. An diese Phase kann sich eine Trockenphase anschließen, in der sich in der Sterilisationskammer 10 vorzugsweise Vakuum befindet.

Figur 3 zeigt ein Schlauchsystem 300 bzw. ein sogenanntes Schlauchset 300, das mittels der in Figur 1 und 2 gezeigten Vorrichtung und Verfahren sterilisiert wurde. Es handelt sich um ein Schlauchset 300 für die Dialyse, das je nach Behandlungsverfahren eine gesamte Schlauchlänge von bis zu 6 m haben kann.

Nicht dargestellt ist die Verpackung für das Schlauchset 300, in der das Schlauchset 300 jedoch vorzugsweise sterilisiert wird. Denkbar ist, dass in einem derartigen Fall wenigstens ein Stützdruck oberhalb des Atmosphärendrucks liegt bzw. angelegt wird.

Das Schlauchset 300 gemäß Figur 3 ist ein Schlauchset 300 für den extrakorporalen Blutkreislauf, das insbesondere eine Nadel 302 zum Anschluss des Schlauchsets 300 z. B. an den Shunt des Patienten, einen Konnektor 304 z. B. zum Anschluss an den nicht dargestellten Dialysator, mit Verschlusskappen verschlossene Zu- bzw. Abläufe 310, 312, 314, 316 sowie Druckmesseranschlüsse 320, 322 aufweist.

Die mit B1 gekennzeichneten Bereiche werden durch das Anlegen des ersten Stützdruckes von ca. 500 mbar abs sterilisiert, also während der in Figur 2 näher dargestellten Sterilisationsphase S1. Betroffen sind alle Bereiche des Schlauchsets 300, in denen das Lumen des Schlauchssets 300 endseitig verschlossen ist, also die mit Verschlusskappen verschlossene Zu- bzw. Abläufe 310, 312, 314, 316 sowie die Druckmesseranschlüsse 320, 322. Es ist denkbar, dass bei besonders langen Schlauchsets 300 der mittlere Bereich des Schlauchssets 300 ebenfalls während der Sterilisationsphase S1 sterilisiert wird.

Die mit B2 gekennzeichneten Bereiche werden ebenfalls durch das Anlegen des ersten Stützdruckes von ca. 500 mbar abs sterilisiert. Betroffen sind alle Bereiche des Schlauchsets 300, die im inneren Bereich des Lumens des Schlauchsets 300 liegen, so dass der erste Stützdruck erforderlich bzw. ausreichend ist, um dass Sterilisationsmittel hierhin zu transportieren.

Die mit B3 gekennzeichneten Bereiche werden durch das Anlegen des zweiten Stützdruckes bzw. Begasungsdruckes von ca. 50 mbar abs sterilisiert, also während der in Figur 2 näher dargestellten Sterilisationsphase S2. Diese Bereiche sind hier in Figur 3 die Anfangs- bzw. Endbereiche des Schlauchssets 300, also die Bereiche, die sich an die Nadel 302 bzw. den Anschluss 304 anschließen.

## Patentansprüche

1. Verfahren zur Sterilisation wenigstens eines medizinischen Schlauchsets (300), wobei das Schlauchset (300) einem Sterilisationsmittel ausgesetzt wird, wobei wenigstens ein erster und wenigstens ein zweiter Stützdruck angelegt werden, **dadurch gekennzeichnet, dass** durch das Sterilisationsmittel, das zumindest teilweise Ozon und/oder Wasserstoffperoxid enthält, bei dem ersten Stützdruck eine Sterilisierung wenigstens eines ersten Bereiches (B1) des Schlauchsets (300) und bei dem zweiten, zu dem ersten Stützdruck unterschiedlichen Stützdruck eine Sterilisierung wenigstens eines zweiten Bereiches (B2) des Schlauchsets (300) erfolgt, wobei der wenigstens eine erste und der wenigstens eine zweite Stützdruck durch Einleitung eines Stützgases in eine Sterilisationskammer (10) erzeugt wird und für eine Zeitspanne im Minutenbereich gehalten wird, wobei das Stützgas zumindest teilweise ein Inertgas und/oder Luft enthält und wobei der erste und der zweite Stützdruck unter Atmosphärendruck liegen und der erste Stützdruck mindestens 500 mbar abs und der zweite Stützdruck mindestens 50 mbar abs beträgt und wobei das Einführen eines Sterilisationsmittels in die Sterilisationskammer (10) bei einem Druck in der Sterilisationskammer (10) erfolgt, der unterhalb des Atmosphärendruckes liegt.

2. Verfahren nach einem der vorhergehenden Ansprüche, **dadurch gekennzeichnet, dass** ein dritter Stützdruck angelegt wird, wobei der dritte Stützdruck vorzugsweise bis zu 50 mbar abs beträgt und/oder dem Begasungsdruck ohne zusätzliches Einleiten eines Stützgases entspricht.

3. Sterilisationsvorrichtung mit wenigstens einer Sterilisationskammer (10) für die Aufnahme wenigstens eines zu sterilisierenden Schlauchsets (300), mit wenigstens einem ersten Mittel zur Zuführung und/oder Abführung eines Sterilisationsmittels, mit wenigstens einem zweiten Mittel, mittels dessen wenigstens ein erster und wenigstens ein zweiter Stützdruck anlegbar ist und wobei bei dem ersten Stützdruck das Sterilisationsmittel eine Sterilisierung eines ersten Bereiches (B1) des Schlauchsets (300) durchführbar und bei dem zweiten Stützdruck eine Sterilisierung eines zweiten Bereiches (B2) des Schlauchsets (300) durchführbar ist, **dadurch gekennzeichnet, dass** die Sterilisationsvorrichtung wenigstens ein Steuerungsmittel aufweist, mittels dessen der Sterilisationsvorgang steuerbar ist, wobei das Steuerungsmittel so ausgeführt ist, dass ein Verfahren nach einem der Ansprüche 1 bis 2 durchführbar ist.

## Claims

1. A method for sterilizing at least one medical hose kit (300), wherein the hose kit (300) is exposed to a sterilization agent, wherein at least one first support pressure and at least one second support pressure are applied, **characterized in that** a sterilization of at least one first region (B1) of the hose kit (300) takes place by the sterilization agent including at least partially ozone and/or hydrogen peroxide at the first support pressure and a sterilization of at least one second region (B2) of the hose kit (300) takes place at the second support pressure differing from the first support pressure, wherein the at least one first support pressure and the at least one second support pressure are generated by introducing a support gas into a sterilization chamber (10) and are held for a duration whose length is in the range of minutes, wherein the support gas includes at least partially an inert gas and/or air and wherein the first support pressure and the second support pressure are below the atmospheric pressure and the first support pressure is at least 500 mbar abs and the second support pressure is at least 50 mbar abs and wherein the introduction of a sterilization agent into the sterilization chamber (10) takes place at a pressure in the sterilization chamber (10) which is below the atmospheric pressure.

2. A method in accordance with one of the preceding claims, **characterized in that** a third support pressure is applied, wherein the third support pressure is preferably up to 50 mbar abs and/or corresponds to the gassing pressure without additional introduction of a support gas.

3. A sterilization apparatus having at least one sterilization chamber (10) for the reception of at least one hose kit (300) to be sterilized, having at least one first means for the supply and/or removal of a sterilization agent, having at least one second means by means of which at least one first support pressure and at least one second support pressure can be applied, and wherein at the first support pressure of the sterilization agent a sterilization of a first region (B1) of the hose kit (300) can be carried out and at a second support pressure a sterilization of a second region (B2) of the hose kit (300) can be carried out, **characterized in that** the sterilization apparatus has at least one control means by means of which the sterilization procedure can be controlled, wherein the control means is configured such that a method in accordance with one of the claims 1 to 2 can be carried out.

## Revendications

1. Procédé de stérilisation d'au moins un ensemble de tuyaux (300) médical, l'ensemble de tuyaux (300) étant exposé à un agent de stérilisation, au moins une première et au moins une deuxième pression d'appui étant appliquées, **caractérisé en ce que**, au moyen de l'agent de stérilisation, qui contient au moins en partie de l'ozone et/ou du peroxyde d'hydrogène, une stérilisation d'au moins une première zone (B1) de l'ensemble de tuyaux (300) se fait à la première pression d'appui et une stérilisation d'au moins une deuxième zone (B2) de l'ensemble de tuyaux (300) à la deuxième pression d'appui, différente de la première pression d'appui, l'au moins une première et l'au moins une deuxième pression d'appui étant générées par l'introduction d'un gaz d'appui dans une chambre de stérilisation (10) et étant maintenues pendant un laps de temps de l'ordre de la minute, le gaz d'appui contenant au moins en partie un gaz inerte et/ou de l'air et la première et la deuxième pression d'appui étant inférieures à la pression atmosphérique et la première pression d'appui étant d'au moins 500 mbar abs et la deuxième pression d'appui d'au moins 50 mbar abs et l'introduction d'un agent de stérilisation dans la chambre de stérilisation (10) se faisant à une pression dans la chambre de stérilisation (10) qui est inférieure à la pression atmosphérique.

2. Procédé selon l'une des revendications précédentes, **caractérisé en ce qu'**une troisième pression d'appui est appliquée, la troisième pression d'appui étant de préférence de 50 mbar abs maximum et/ou correspondant à la pression du gazage sans introduction supplémentaire d'un gaz d'appui.

3. Dispositif de stérilisation comprenant au moins une chambre de stérilisation (10) destinée à recevoir au moins un ensemble de tuyaux (300) à stériliser, comprenant au moins un premier moyen destiné à alimenter et/ou évacuer un agent de stérilisation, au moins un second moyen, à l'aide duquel au moins une première et au moins une deuxième pression d'appui peuvent être appliquées et dans lequel, au moyen de l'agent de stérilisation, une stérilisation d'une première zone (B1) de l'ensemble de tuyaux (300) peut être effectuée à la première pression d'appui et une stérilisation d'une seconde zone (B2) de l'ensemble de tuyaux (300) peut être effectuée à la deuxième pression d'appui, **caractérisé en ce que** le dispositif de stérilisation comporte au moins un moyen de commande, à l'aide duquel le processus de stérilisation peut être commandé, le moyen de commande étant conçu de telle manière qu'un procédé selon l'une des revendications 1 à 2 peut être exécuté.
